# EUROPEAN PATENT APPLICATION

(11) **EP 3 593 720 A1**
(43) Date of publication of application: **15.01.2020**
(21) Application number: 17899795.3
(22) Date of filing: 09.03.2017
(51) Int. Cl.: A61B 5/20

(54) **URINATION INFORMATION DISPLAY DEVICE AND URINATION INFORMATION DISPLAY SYSTEM**

(71) Applicant: Shimadzu Corporation, Kyoto-shi, Kyoto 604-8511 (JP)
(72) Inventor: HASHIZUME, Nobuya, Kyoto-shi Kyoto 604-8511 (JP); SHIMIZU, Kazunori, Kyoto-shi Kyoto 604-8511 (JP)
(74) Representative: Kilian Kilian & Partner
(86) International application number: PCT/JP2017/009590
(87) International publication number: WO 2018/163379

(57) **Abstract**

This voiding data display device (100) includes a voiding data display controller (5a) configured or programmed to display voiding data including a voiding date and time and a voided volume, a time data display controller (5b) configured or programmed to display data on a wake-up time and data on a fall-asleep time, and an analysis result display controller (5c) configured or programmed to display a result of analysis of the voiding data, and the voiding data display device is configured to display the voiding data, the data on the wake-up time, the data on the fall-asleep time, and the result of the analysis of the voiding data on a same window.

## Description

### Technical Field

The present invention relates to a voiding data display device, and more particularly, it relates to a voiding data display device and a voiding data display system, both of which are capable of computerizing and displaying patient's voiding data.

### Background Art

Conventionally, a voiding data display device capable of computerizing and displaying patient's voiding data is known. Such a voiding data display device is disclosed in Japanese Patent Laid-Open No. 2006-231032, for example.

Japanese Patent Laid-Open No. 2006-231032 discloses a voiding data display device capable of saving a patient the hassle of manually filling in a voiding form, capable of ensuring the accuracy of a voiding record, and capable of saving a doctor the hassle of manually entering data into a computer. In the voiding data display device disclosed in Japanese Patent Laid-Open No. 2006-231032, the patient provides voiding data measured by a voided volume measurement device (electronic balance) by storing the voiding data on a server installed in a hospital through wireless communication or giving the doctor a storage medium that records the voiding data. When checking the voiding data, the doctor acquires the patient's voiding data by accessing the server installed in the hospital from the computer or reading the voiding data from the storage medium given by the patient, and displays the acquired patient's voiding data in a time sequence on the voiding data display device.

### Prior Art

### Patent Document

Patent Document 1: Japanese Patent Laid-Open No. 2006-231032

### Summary of the Invention

### Problem to be Solved by the Invention

However, the voiding data display device disclosed in Japanese Patent Laid-Open No. 2006-231032 only displays patient's voiding data in a time sequence. Therefore, when using the patient's voiding data for diagnosis, it is difficult for the doctor to link and grasp patient's lifestyle habits, such as waking up and falling asleep, and the voiding data at first glance.

The present invention is intended to solve the above problem. The present invention aims to provide a voiding data display device and a voiding data display system, both of which are configured to enable a doctor to link patient's lifestyle habits, such as waking up and falling asleep, to patient's voiding data and to easily grasp the same when diagnosing the patient's voiding data. Means for Solving the Problem

In order to attain the aforementioned object, a voiding data display device according to a first aspect of the present invention includes a voiding data display controller configured or programmed to display voiding data including a voiding date and time and a voided volume, a time data display controller configured or programmed to display data on a wake-up time and data on a fall-asleep time, and an analysis result display controller configured or programmed to display a result of analysis of the voiding data. Furthermore, the voiding data display device is configured to display the voiding data, the data on the wake-up time, the data on the fall-asleep time, and the result of the analysis of the voiding data on a same window.

As described above, the voiding data display device according to the first aspect of the present invention includes the voiding data display controller configured or programmed to display the voiding data, the time data display controller configured or programmed to display the data on the wake-up time and the data on the fall-asleep time, and the analysis result display controller configured or programmed to display the result of the analysis of the voiding data, and the voiding data display device is configured to display the voiding data, the data on the wake-up time, the data on the fall-asleep time, and the result of the analysis of the voiding data on the same window. Accordingly, data necessary for diagnosis, such as the voiding data, the data on the wake-up time, the data on the fall-asleep time, and the result of the analysis of the voiding data, can be displayed in a display mode of arrangement on the same window. Therefore, it is not necessary for a doctor to compare the voiding data arranged in a time sequence and an analysis result obtained by separately analyzing the voiding data. Consequently, when diagnosing patient's voiding data, the doctor can link patient's lifestyle habits, such as waking up and falling asleep, to the patient's voiding data and easily grasp the same.

The aforementioned voiding data display device according to the first aspect is preferably configured to visually distinguishably display a first voiding data logged from the wake-up time to the fall-asleep time and a second voiding data logged from the fall-asleep time to the wake-up time in different display modes. According to this configuration, the voiding data during waking up can be visually distinguished from the voiding data during falling asleep, and thus the doctor can determine whether the voiding data is data during waking up or data during falling asleep at a glance without comparing a voiding time with the wake-up time and the fall-asleep time.

In this case, the voiding data display device is preferably configured to display the first voiding data logged from the wake-up time to the fall-asleep time and the second voiding data logged from the fall-asleep time to the wake-up time in character colors different from each other or background colors different from each other. According to this configuration, the first voiding data logged from the wake-up time to the fall-asleep time and the second voiding data logged from the fall-asleep time to the wake-up time are color-coded, and thus it is possible to more easily distinguish between the voiding data during waking up and the voiding data during falling asleep.

The aforementioned voiding data display device according to the first aspect preferably includes a first display area arranged for displaying data during a unit time period including the voiding data, the data on the wake-up time, the data on the fall-asleep time, and the result of the analysis of the voiding data is displayed, and a second display area arranged for displaying at least one of the data included in the first display area is displayed over a plurality of unit time periods. According to this configuration, for example, the presence or absence of a sudden, abnormal change in a short period of time can be checked from the data during the unit time period including the voiding data, the data on the wake-up time, the data on the fall-asleep time, and the result of the analysis of the voiding data. In addition, long-term progress such as the progress of treatment can be checked from the data over the plurality of unit time periods including the voiding data, the data on the wake-up time, the data on the fall-asleep time, and the result of the analysis of the voiding data.

In this case, the first display area is preferably arranged for displaying data for one day as the unit time period in a time sequence, and the second display area is preferably arranged for graphically displaying data for multiple days. According to this configuration, in the first display area, actual data in a time sequence of one day can be checked, and thus an abnormal change or the like in one day can be checked in more detail. Moreover, in the second display area, the data for multiple days can be graphically and visually checked, and thus the progress of treatment or the like can be more easily grasped.

The aforementioned configuration including the first display area and the second display area is preferably configured to display the first display area and the second display area on different windows. According to this configuration, too much stuffing of information on one window can be significantly reduced or prevented, and thus it is possible to make the voiding data easy to see.

In the aforementioned voiding data display device according to the first aspect, the result of the analysis of the voiding data preferably includes at least one of a total voided volume, a total daily voiding frequency, and a set of an average voided volume during waking up and an average voided volume during falling asleep and/or a set of a voiding frequency during waking up and a voiding frequency during falling asleep. According to this configuration, the result of the analysis of the voiding data necessary for diagnosis, such as the total voided volume, the total daily voiding frequency, and the set of the average voided volume during waking up and the average voided volume during falling asleep and/or the set of the voiding frequency during waking up and the voiding frequency during falling asleep, can be checked along with the voiding data, the data on the wake-up time, and the data on the fall-asleep time, and thus the burden of diagnosis can be reduced.

The aforementioned voiding data display device according to the first aspect preferably further includes an additional information display controller configured or programmed to display additional information of the voiding data including at least one of urinary urgency, urine leakage, and a presence or absence of defecation. According to this configuration, the situation at the time of voiding can be grasped in more detail, and thus the accuracy of diagnosis can be improved.

In the aforementioned voiding data display device according to the first aspect, the time data display controller preferably includes a wake-up and fall-asleep indication display controller configured or programmed to display, between the voiding data arranged in a time sequence, a wake-up time indication and a fall-asleep time indication as the data on the wake-up time and the data on the fall-asleep time, respectively. According to this configuration, the wake-up time and the fall-asleep time can be visually grasped through the wake-up time indication and the fall-asleep time indication, and thus it is possible to easily distinguish between the range of the voiding data during waking up and the range of the voiding data during falling asleep among the voiding data arranged in a time sequence.

The aforementioned voiding data display device according to the first aspect preferably further includes a highlighting controller configured or programmed to display an indication that highlights numerical data exceeding a threshold among at least one of the voiding data and the result of the analysis of the voiding data. According to this configuration, an oversight of data indicating an abnormal value (a value exceeding the threshold set by the doctor) can be significantly reduced or prevented.

In this case, the voiding data display device preferably further includes a threshold input receiver configured to receive an input of the threshold, and the highlighting controller is preferably configured or programmed to be able to change the threshold upon the received input of the threshold. According to this configuration, the doctor as a user can freely set an appropriate threshold, and thus the convenience of diagnosis in accordance with the criteria different for each doctor can be improved.

The aforementioned voiding data display device according to the first aspect preferably further includes a wake-up and fall-asleep time input receiver configured to receive the data on the wake-up time and the data on the fall-asleep time, and a wake-up and fall-asleep time modifier configured to modify the data on the wake-up time and the data on the fall-asleep time based on input data acquired by the wake-up and fall-asleep time receiver. According to this configuration, the data on the wake-up time and the data on the fall-asleep time can be automatically acquired. When the acquired data is incorrect, it can be modified. Consequently, it is possible to avoid making a diagnosis using erroneous data, and thus the accuracy of the diagnosis can be improved.

A voiding data display system according to a second aspect of the present invention includes a voiding data display device including a voiding data display controller configured or programmed to display voiding data including a voiding date and time and a voided volume, a time data display controller configured or programmed to display data on a wake-up time and data on a fall-asleep time, and an analysis result display controller configured or programmed to display a result of analysis of the voiding data, and a voided volume measurement device connectable to the voiding data display device wirelessly or by wire. Furthermore, the voiding data display device is configured to acquire at least one of the voiding data and a set of the data on the wake-up time and the data on the fall-asleep time from the voided volume measurement device.

As described above, the voiding data display system according to the second aspect of the present invention includes the voiding data display device including the voiding data display controller configured or programmed to display the voiding data, the time data display controller configured or programmed to display the data on the wake-up time and data on the fall-asleep time, and the analysis result display controller configured or programmed to display the result of the analysis of the voiding data, and the voided volume measurement device. Furthermore, the voiding data display device is configured to acquire at least one of the voiding data and the set of the data on the wake-up time and the data on the fall-asleep time from the voided volume measurement device. Accordingly, the voiding data display device can acquire data necessary for a doctor to make a diagnosis from the voided volume measurement device, and display the voiding data, the data on the wake-up time, the data on the fall-asleep time, and the result of the analysis of the voiding data. Consequently, when diagnosing patient's voiding data, the doctor can link patient's lifestyle habits, such as waking up and falling asleep, to the patient's voiding data and easily grasp the same. In addition, the voiding data can be acquired from the voided volume measurement device, and thus it is possible to save the doctor the hassle of manual entry. Furthermore, analyzed data can be acquired, and thus it is possible to save the doctor the hassle of analysis.

### Effect of the Invention

According to the present invention, as described above, it is possible to provide the voiding data display device and the voiding data display system, both of which are configured to enable the doctor to link the patient's lifestyle habits, such as waking up and falling asleep, to the patient's voiding data and to easily grasp the same when diagnosing the patient's voiding data.

### Brief Description of the Drawings

FIG. 1 is a schematic view of the overall configuration of a voiding data display system including a voiding data display device according to an embodiment of the present invention.
FIG. 2 is a block diagram schematically showing the voiding data display device according to the embodiment of the present invention.
FIG. 3 is an image diagram of a voiding diary display window of the voiding data display device according to the embodiment of the present invention.
FIG. 4 is an image diagram (A) before modification, an image diagram (B) during operation, and an image diagram (C) after modification for illustrating the modification of a wake-up time of a voiding diary in the voiding data display device according to the embodiment of the present invention.
FIG. 5 is an image diagram (A) before modification, an image diagram (B) during operation, and an image diagram (C) after modification for illustrating the modification of a fall-asleep time of the voiding diary in the voiding data display device according to the embodiment of the present invention.
FIG. 6 is an image diagram of a day and night voiding frequency display window of the voiding data display device according to the embodiment of the present invention.
FIG. 7 is an image diagram of a display window of total voided volumes during a predetermined period of the voiding data display device according to the embodiment of the present invention.
FIG. 8 is an image diagram of a display window of voided volumes during the predetermined period of the voiding data display device according to the embodiment of the present invention.
FIG. 9 is an image diagram of a threshold setting window, which is configured to set a threshold, of the voiding data display device according to the embodiment of the present invention.
FIG. 10 is an image diagram of a daily voided volume display window of a voiding data display device according to a first modified example of the embodiment of the present invention.
FIG. 11 is an image diagram of a display window of statistical data during the period of a voiding data display device according to a second modified example of the embodiment of the present invention.
FIG. 12 is an image diagram of a voiding diary display window of a voiding data display device according to a third modified example of the embodiment of the present invention.
FIG. 13 is an image diagram of a voiding diary display window of a voiding data display device according to a fourth modified example of the embodiment of the present invention.
FIG. 14 is an image diagram of a voiding diary display window of a voiding data display device according to a fifth modified example of the embodiment of the present invention.

### Modes for Carrying Out the Invention

An embodiment embodying the present invention is hereinafter described on the basis of the drawings.

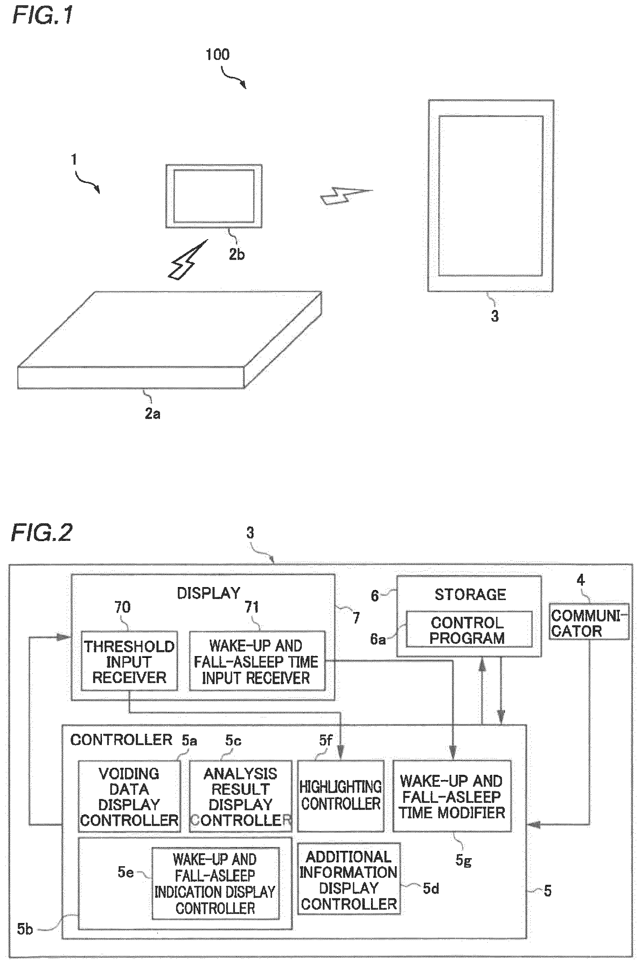

The configuration of a voiding data display system 100 including a voiding data display device 3 according to the embodiment is now described with reference to FIGS. 1 to 11.

### (Configuration of Voiding Data Display System)

As shown in FIG. 1, the voiding data display system 100 according to this embodiment includes a voided volume measurement device 1 and a voiding data display device 3. In an example shown in FIG. 1, the voided volume measurement device 1 includes a weight scale 2a and a subject terminal 2b, for example, and is configured to measure the weight of a subject before and after voiding with the weight scale 2a and to transmit the same to the subject terminal 2b.

The subject terminal 2b is configured to calculate a voided volume from the weight before and after voiding transmitted from the weight scale 2a, and to store voiding data including the calculated voided volume, and a voiding date and time. In addition, the subject terminal 2b is configured to be capable of receiving additional information such as urgency at the time of voiding, urine leakage, the presence or absence of defecation from the subject. The subject terminal 2b is configured to be wirelessly connectable to the voiding data display device 3, and to transmit the voiding data, data on a wake-up time, data on a fall-asleep time, the additional information, etc. to the voiding data display device 3. The subject terminal 2b is a tablet terminal, for example.

The voiding data display device 3 is configured to analyze the voiding data, the data on a wake-up time, the data on a fall-asleep time, and the additional information transmitted from the subject terminal 2b, and to perform display such that a doctor can easily grasp the patient's condition at a glance.

### (Configuration of Voiding Data Display Device)

As shown in FIG. 2, the voiding data display device 3 according to this embodiment includes a communicator 4, a controller 5, a storage 6, and a display 7. The voiding data display device 3 is a tablet terminal, for example.

The communicator 4 communicates with an external device to transmit and receive information. In this embodiment, the communicator 4 is configured to receive the voiding data, the data on a wake-up time, the data on a fall-asleep time, and the additional information from the subject terminal 2b and transmit the same to the controller 5.

The controller 5 includes a processor such as a central processing unit (CPU), for example. The CPU functions as a controller configured or programmed to control the voiding data display device 3 by executing a control program 6a. The controller 5 includes a voiding data display controller 5a, a time data display controller 5b, an analysis result display controller 5c, an additional information display controller 5d, a wake-up and fall-asleep indication display controller 5e, a highlighting controller 5f, and a wake-up and fall-asleep time modifier 5g as functions by executing the control program 6a.

The voiding data display controller 5a is configured or programmed to arrange the voiding data including a voiding date and time and a voided volume acquired from the communicator 4 in a time sequence and display the same in a tabular form.

The time data display controller 5b is configured or programmed to display the data on a wake-up time and the data on a fall-asleep time between the voiding data arranged in a time sequence.

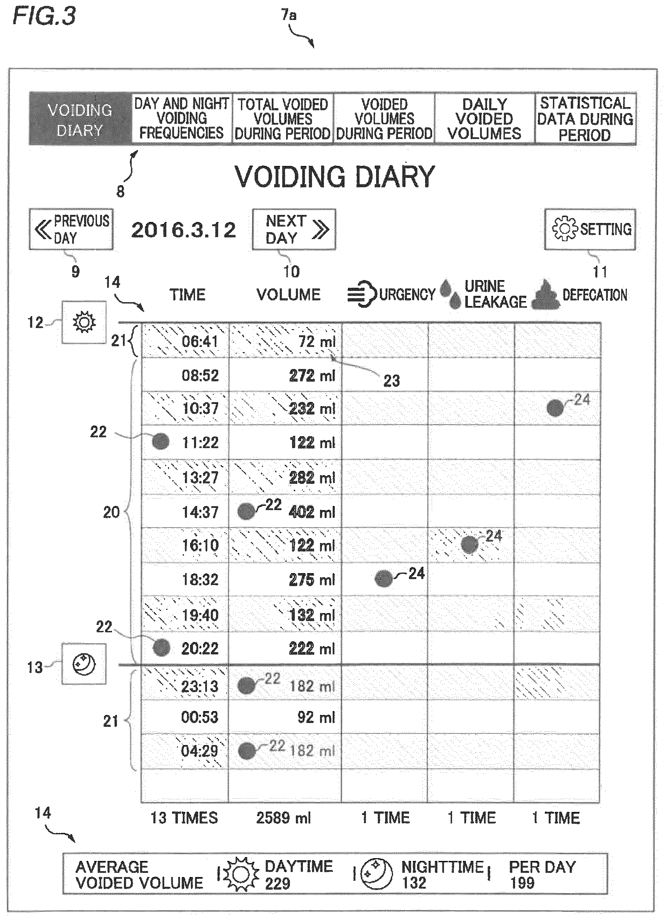

The time data display controller 5b includes the wake-up and fall-asleep indication display controller 5e configured or programmed to display, between the voiding data arranged in a time sequence, a wake-up time indication 12 (see FIG. 3) and a fall-asleep time indication 13 (FIG. 3) as the data on a wake-up time and the data on a fall-asleep time, respectively.

The analysis result display controller 5c is configured or programmed to display results of analysis of the voiding data. Specifically, the analysis result display controller 5c is configured or programmed to calculate and display a daily voiding frequency and a daily voided volume. Furthermore, the analysis result display controller 5c is configured or programmed to calculate and display an average value of voided volumes during waking up, an average value of voided volumes during falling asleep, and an average value of daily voided volumes. The analysis result display controller 5c is configured or programmed to calculate and display the number of times of additional information described below. In addition, the analysis result display controller 5c is configured or programmed to graph and display the results of analysis of the voiding data. In this specification, the results of analysis indicate data obtained by calculating and aggregating the voiding data, the data on a wake-up time and the data on a fall-asleep time, and the additional information described below.

The additional information display controller 5d is configured or programmed to display the additional information of the voiding data including urinary urgency (a sudden and unbearable desire to void), urine leakage, and the presence or absence of defecation. For example, in this embodiment, the additional information display controller 5d is configured or programmed to display urgency, urine leakage, and the presence or absence of defecation as the additional information as well as the voiding data displayed in a time sequence. The additional information is subjective or objective information about voiding other than a voided volume obtained at the time of voiding, a voiding date and time, a wake-up time, and a fall-asleep time.

The highlighting controller 5f is configured or programmed to display an indication 22 (see FIG. 3) that highlights numerical data exceeding a threshold among the voiding data and the results of analysis. Specifically, the highlighting controller 5f is configured or programmed to display the indication 22 (see FIG. 3) that highlights numerical data next to a voided volume when there is a voided volume exceeding a voided volume set by the doctor. In addition, the highlighting controller 5f is configured or programmed to display the indication 22 (see FIG. 3) that highlights numerical data next to a voiding time when voiding occurs in a time shorter than a voiding interval set by the doctor.

The wake-up and fall-asleep time modifier 5g is configured to be able to modify the data on a wake-up time and the data on a fall-asleep time. Specifically, the wake-up and fall-asleep time modifier 5g is configured to modify the data on a wake-up time and the data on a fall-asleep time based on data on a wake-up time and data on a fall-asleep time acquired by a wake-up and fall-asleep time input receiver 71.

The storage 6 includes a storage such as a semiconductor storage element or an HDD, and stores the control program 6a for the controller 5 to analyze the voiding data, for example.

The display 7 includes a display such as a liquid crystal monitor or an organic EL monitor, and displays the voiding data, the data on a wake-up time, the data on a fall-asleep time, and the additional information sent from the controller 5. Furthermore, the display 7 includes, as software, a threshold input receiver 70 configured to receive a threshold input by a user (such as a doctor). In addition, the display 7 includes, as software, the wake-up and fall-asleep time input receiver 71 configured to receive inputs of the data on a wake-up time and the data on a fall-asleep time by the user.

The voiding data, the data on a wake-up time, the data on a fall-asleep time, and the additional information received from the subject terminal 2b via the communicator 4 are sent to the controller 5. The controller 5 calls the control program 6a from the storage 6, and analyzes the voiding data, the data on a wake-up time, the data on a fall-asleep time, and the additional information sent via the communicator 4. The controller 5 sends the voiding data, the data on a wake-up time, the data on a fall-asleep time, the results of analysis, and the additional information to the display 7. In addition, the controller 5 controls the display 7 to display the sent voiding data, data on a wake-up time, data on a fall-asleep time, results of analysis, and additional information on the same window. Information input to the threshold input receiver 70 and the wake-up and fall-asleep time input receiver 71 of the display 7 is sent to the highlighting controller 5f and the wake-up and fall-asleep time modifier 5g, respectively. The highlighting controller 5f is configured or programmed to be able to change a threshold upon a received threshold input. The wake-up and fall-asleep time modifier 5g is configured to modify the data on a wake-up time and the data on a fall-asleep time based on the input data acquired by the wake-up and fall-asleep time input receiver 71.

The voiding data display controller 5a displays a voiding date and time and a voided volume in a tabular form in a time sequence. The wake-up and fall-asleep indication display controller 5e included in the time data display controller 5b displays, between the voiding data arranged in a time sequence, the wake-up time indication 12 (see FIG. 3) and the fall-asleep time indication 13 (FIG. 3) as the data on a wake-up time and the data on a fall-asleep time, respectively. The analysis result display controller 5c displays the results of analysis of the voiding data, the data on a wake-up time, the data on a fall-asleep time, and the additional information.

The additional information display controller 5d displays the additional information by displaying indications 24 (see FIG. 3) for urgency, urine leakage, and the presence or absence of defecation next to the voiding data displayed in a time sequence. The highlighting controller 5f highlights data by displaying the indications 22 for data (a voided volume and a voiding interval) exceeding thresholds (upper limit values) set by the doctor.

Windows displayed on the display 7 of the voiding data display device 3 are now specifically described with reference to FIGS. 3 to 11.

FIG. 3 is an image diagram of a voiding diary window 7a displayed on the display 7. On the voiding diary window 7a, a window switching tab 8, a previous day button 9, a next day button 10, a setting button 11, the wake-up time indication 12, the fall-asleep time indication 13, and a first display area 14 including the voiding data during a unit time period, the data on a wake-up time, the data on a fall-asleep time, the results of analysis of the voiding data, and the additional information.

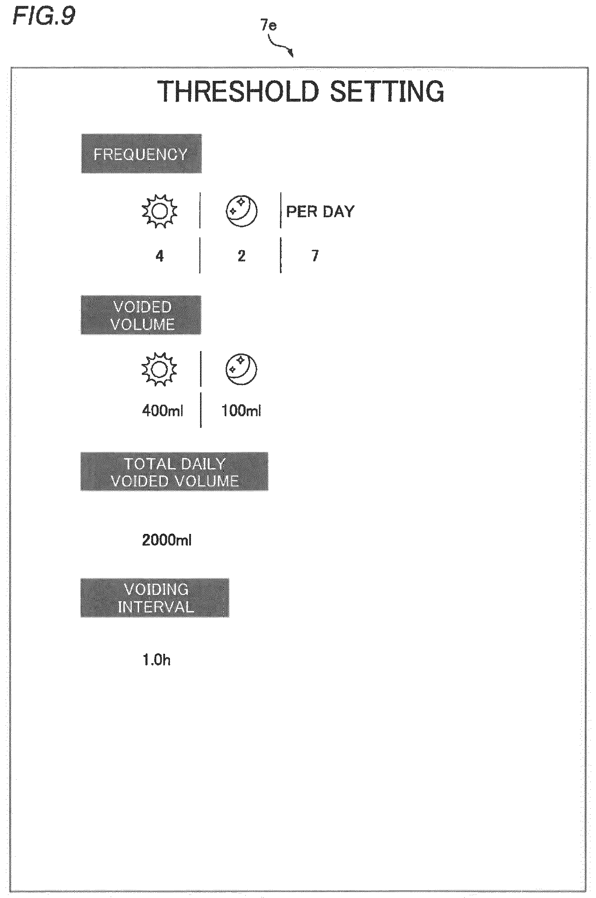

The window switching tab 8 is an input unit configured to allow the user to switch a display window by performing an input operation (a tap, for example). The previous day button 9 and the next day button 10 are buttons configured to allow the user to switch a voiding diary to be displayed (to display a voiding diary on a different day) by performing an input operation (such as a tap). The setting button 11 is a button that takes the user to a threshold setting window 7e (see FIG. 9) configured to allow the user to change a threshold by an input operation (such as tapping).

In the first display area 14, a table showing voiding data including a voiding date and time and a voided volume in a time sequence, an average voided volume during waking up, an average voided volume during falling asleep, and an average daily voided volume are displayed. In addition, the wake-up time indication 12 and the fall-asleep time indication 13 are respectively displayed as the data on a wake-up time and the data on a fall-asleep time between the voiding data arranged in a time sequence. Furthermore, next to the voiding data, urgency at the time of voiding, urine leakage, the presence or absence of defecation are displayed as the additional information. Moreover, the wake-up time indication 12 includes a mark of the sun and a horizontal straight line displayed beside the mark, and the fall-asleep time indication 13 includes a mark of the moon and a horizontal straight line displayed beside the mark.

Among the voiding data, voiding data, such as a voided volume and a voiding interval, exceeding a threshold set on the threshold setting window 7e (see FIG. 9) is displayed and highlighted with the indication 22.

It is difficult to distinguish between the voiding data during waking up and the voiding date during falling asleep at first glance only by arranging the voiding data, the data on a wake-up time, and the data on a fall-asleep time in a time sequence, and it is necessary to compare a wake-up time and a fall-asleep time with each voiding date and time and determine whether the voiding data is data during waking up or data during falling asleep.

Therefore, the voiding data display device 3 is configured to visually distinguishably display a first voiding data logged from the wake-up time to the fall-asleep time and a second voiding data logged from the fall-asleep time to the wake-up time in different display modes on the voiding diary window 7a. FIG. 3 shows an example in which the first voiding data logged from the wake-up time to the fall-asleep time and the second voiding data logged from the fall-asleep time to the wake-up time are displayed in character colors different from each other. For example, the voiding data (voided volumes) in an area 20 representing "during waking up" from the wake-up time indication 12 to the fall-asleep time indication 13 is displayed in orange, and the remaining voiding data (voided volumes) in an area 21 representing "during falling asleep" is displayed in blue. In FIG. 3, a difference in the character color of the voiding data is shown by outline characters and black-filled characters for the sake of convenience.

In FIG. 3, voiding data 23 (06: 41) immediately below the wake-up time indication 12 is voiding data after waking up, but is included in the area 21 representing "during falling asleep". This is because in the first voiding after waking up, urine generated at night is voided, and thus it is regarded as voiding during falling asleep even after waking up.

In FIG. 3, the indications 24 indicate the presence or absence of the additional information. Specifically, the indication 24 displayed in a column of urgency indicates that urgency was felt at the time of voiding at that time. In addition, the indication 24 displayed in a column of urine leakage indicates that there was urine leakage at the time of voiding at that time. Moreover, the indication 24 displayed in a column of defecation indicates that there was defecation at the time of voiding at that time.

Below the table showing the voiding data displayed in a time sequence, the results of analysis including a voiding frequency, a total voided volume, and the number of times of the additional information are displayed. Below that, the results of analysis including an average value of voided volumes during waking up, an average value of voided volumes during falling asleep, and an average value of daily voided volumes are displayed.

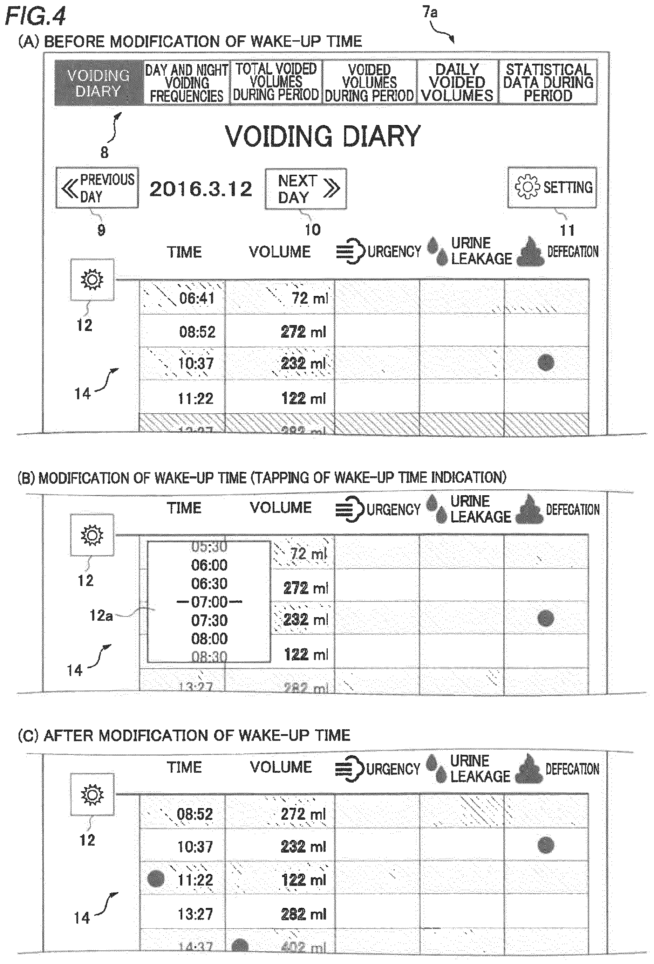

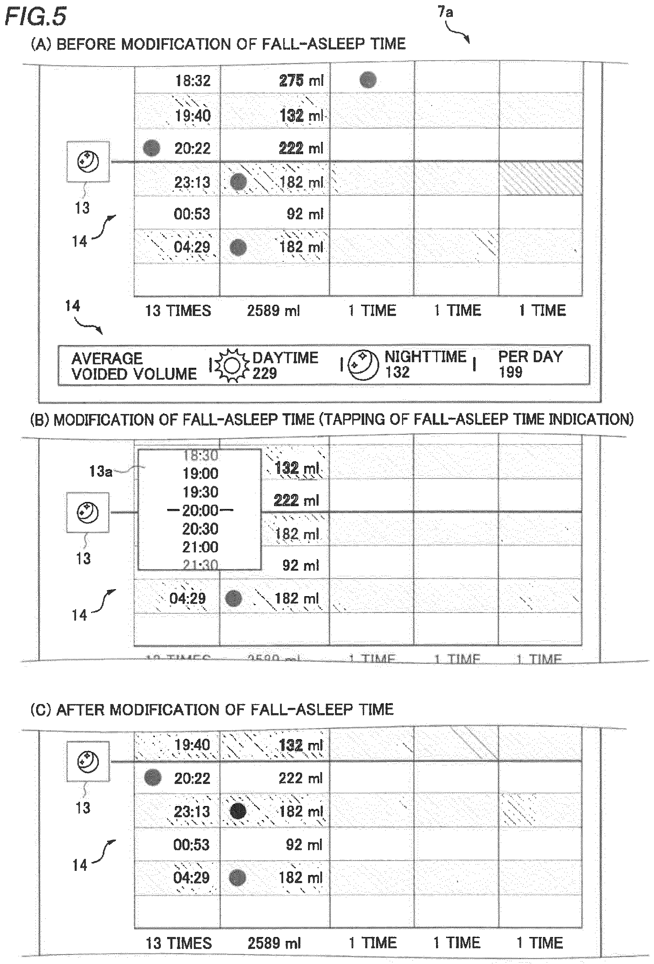

A method for modifying the data on a wake-up time and the data on a fall-asleep time is now described with reference to FIGS. 4 and 5. The data on a wake-up time and the data on a fall-asleep time are data transmitted from the subject terminal 2b (see FIG. 1), and thus normally, it is not necessary for a doctor or the like to input the data. However, when the transmitted data is incorrect or when the data on a wake-up time and the data on a fall-asleep time are not registered in the first place, for example, it may be necessary for the doctor or the like to get the data from a patient and input the same. Therefore, in the voiding data display device 3 according to this embodiment, the wake-up and fall-asleep time modifier 5g modifies the data on a wake-up time and the data on a fall-asleep time based on input data based on input data acquired by the wake-up and fall-asleep time input receiver 71.

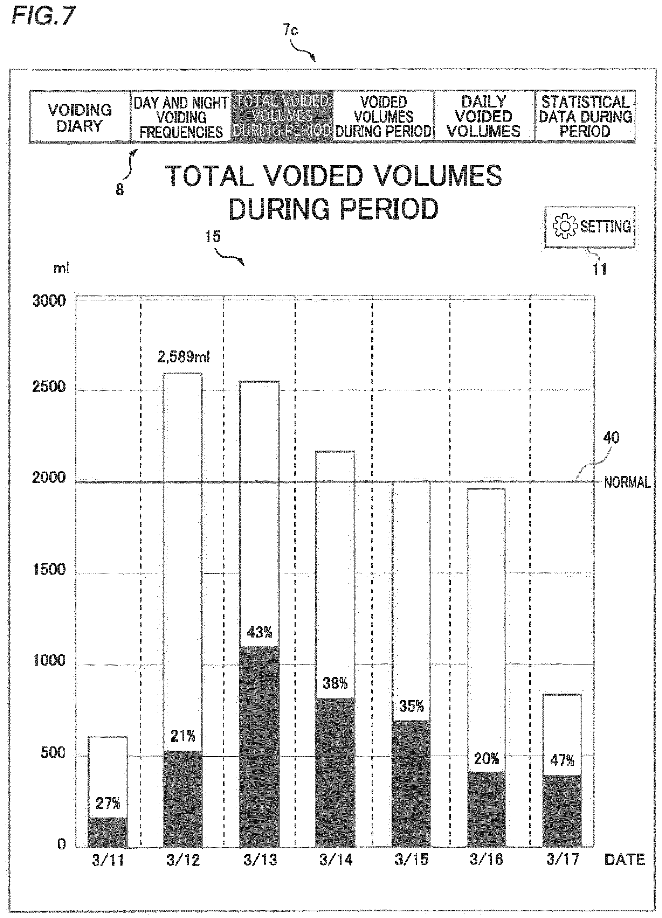

First, a method for modifying the data on a wake-up time is described with reference to FIG. 4. FIG. 4(A) is an image diagram before modification of a wake-up time, and when the user taps the wake-up time indication 12, a wake-up time change list 12a is displayed as the wake-up and fall-asleep time input receiver 71, as shown in FIG. 4(B). When the wake-up time is changed (in FIG. 4(B), 07:00 is selected), the wake-up time is modified by the wake-up and fall-asleep time modifier 5g, and the voiding data displayed in the first display area 14 is changed as shown in FIG. 4(C). That is, when the wake-up time is changed, the voiding data before the wake-up time is included in the voiding data of the previous day, and thus it is no longer displayed in the first display area 14. The wake-up time change list 12a is an example of a "wake-up and fall-asleep time input receiver" in the claims.

Next, a method for modifying the data on a fall-asleep time is described with reference to FIG. 5. FIG. 5(A) is an image diagram before modification of a fall-asleep time, and when the user taps the fall-asleep time indication 13, a fall-asleep time change list 13a is displayed as the wake-up and fall-asleep time input receiver 71, as shown in FIG. 5(B). When the fall-asleep time is changed (in FIG. 5(B), 20:00 is selected), the fall-asleep time is modified by the wake-up and fall-asleep time modifier 5g, and the fall-asleep time indication 13 is moved as shown in FIG. 5(C). The fall-asleep time change list 13a is an example of a "wake-up and fall-asleep time input receiver" in the claims.

The voiding data display device 3 according to this embodiment includes the first display area 14 arranged for displaying data during a unit time period including the voiding data, the data on a wake-up time, the data on a fall-asleep time, and the results of analysis of the voiding data, and a second display area 15 arranged for displaying at least one of the data included in the first display area 14 over a plurality of unit time periods. In this specification, the unit time period is one day, for example.

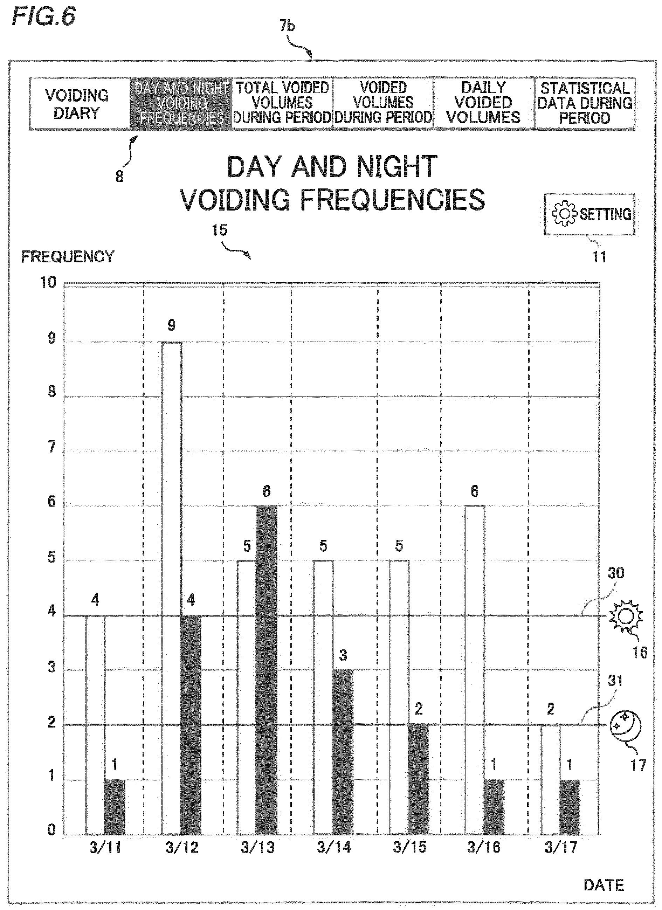

Specifically, in the voiding data display device 3, the first display area 14 is arranged for displaying data for one day as a unit time period in a time sequence, and the second display area 15 is arranged for graphically displaying data for multiple days. The second display area 15 is arranged for displaying day and night voiding frequencies (see FIG. 6), for example. The second display area 15 is also arranged for displaying total voided volumes (see FIG. 7) during the period, for example. Note that in this embodiment, data is displayed while defining one day, which serves as a unit time period, as a period from waking up to waking up the next day.

The contents displayed in the second display area 15 of the voiding data display device 3 according to this embodiment are now described with reference to FIGS. 6 to 8. The second display area 15 of the voiding data display device 3 according to this embodiment is arranged for displaying at least one of the data included in the first display area 14 that spans the plurality of unit time periods. The plurality of unit time periods are one week, for example.

First, an example in which a day and night voiding frequency window 7b is displayed as the second display area 15 is described with reference to FIG. 6.

FIG. 6 is a graphical representation of day and night voiding frequencies over the plurality of unit time periods. The horizontal axis of the graph represents the date, and the vertical axis of the graph represents the voiding frequency. White bars in the graph each indicate a voiding frequency during waking up, and black bars in the graph each indicate a voiding frequency during falling asleep. That is, the voiding frequency during waking up and the voiding frequency during falling asleep are distinguishably displayed. A horizontal straight line 30 on which a during-waking-up indication 16 is displayed is a straight line indicating a threshold (upper limit value) of a voiding frequency during waking up set by the doctor. A straight line 31 on which a during-falling-asleep indication 17 is displayed is a straight line indicating a threshold (upper limit value) of a voiding frequency during falling asleep set by the doctor. Through the day and night voiding frequency window 7b, the doctor can grasp at a glance the transition (change) of the day and night voiding frequencies over the plurality of unit time periods. In addition, the doctor can grasp at a glance the number of days on which or a frequency at which the voiding frequency during waking up and/or the voiding frequency during falling asleep exceeds the threshold.

Next, an example in which a within-period total voided volume window 7c is displayed as the second display area 15 is described with reference to FIG. 7.

FIG. 7 is a graphical representation of total voided volumes over the plurality of unit time periods. The horizontal axis of the graph represents the date, and the vertical axis of the graph represents the voided volume (ml). White bars in the graph each indicate a voided volume during waking up, and black bars in the graph each indicate a voided volume during falling asleep. That is, the voided volume during waking up and the voided volume during falling asleep are distinguishably displayed. A horizontal straight line 40 marked as normal is a straight line indicating a threshold (upper limit value) of a total daily voided volume set by the doctor. Furthermore, numerical values expressed as a percentage in the graph are numerical values indicating the percentages of voided volumes during falling asleep (voided volume during falling asleep /total daily voided volume × 100). When the percentage of a voided volume during falling asleep exceeds 33% (1/3), it can be determined that the voided volume during falling asleep is large. In addition, a numerical value (total voided volume) of the largest daily voided volume during the period is shown in the graph. Through the within-period total voided volume window 7c, the doctor can grasp at a glance the transition (change) of the total voided volumes during the period, the transition (change) of the percentages of the voided volumes during falling asleep, and the number of days (frequency) on which the total daily voided volume exceeds the threshold, for example.

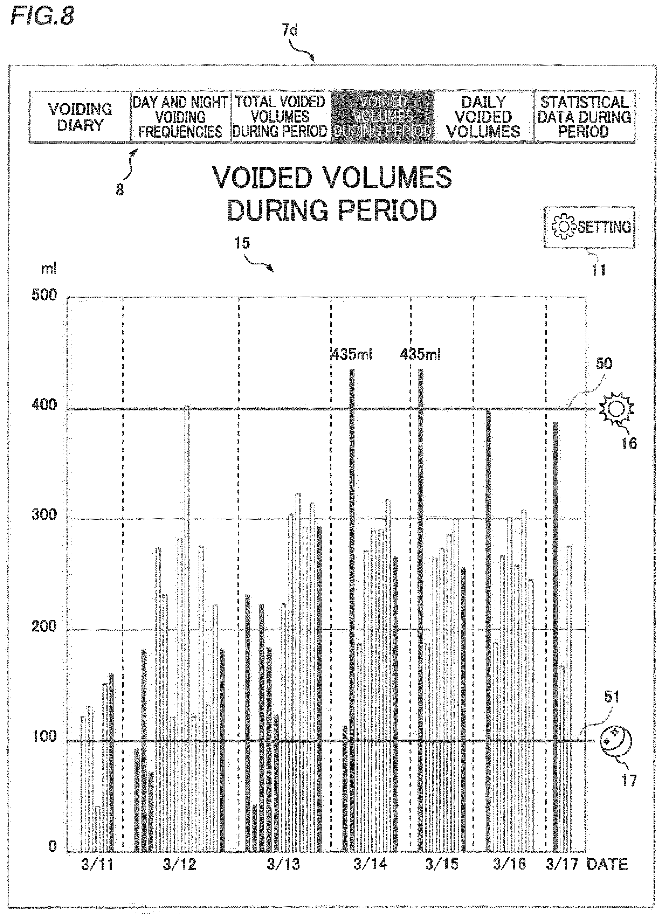

Next, an example in which a within-period voided volume window 7d is displayed as the second display area 15 is described with reference to FIG. 8.

FIG. 8 is a graphical representation of voided volumes over the plurality of unit time periods. The horizontal axis of the graph represents the date, and the vertical axis of the graph represents the voided volume (ml). White bars in the graph each indicate a voided volume during waking up, and black bars in the graph each indicate a voided volume during falling asleep. That is, the voided volume during waking up and the voided volume during falling asleep are distinguishably displayed. A horizontal straight line 50 on which the during-waking-up indication 16 is displayed is a straight line indicating a threshold (upper limit value) of a voided volume during waking up set by the doctor. A horizontal straight line 51 on which the during-falling-asleep indication 17 is displayed is a straight line indicating a threshold (upper limit value) of a voided volume during falling asleep set by the doctor. In addition, a numerical value (one-time voided volume) of the largest voided volume during the period is shown in the graph. Through the within-period voided volume window 7d, the doctor can grasp at a glance the transition (change) of the voiding frequencies of the voiding data over the plurality of unit time periods, the transition (change) of the voided volumes, and the voided volume (one-time voided volume) exceeding the threshold, for example.

The voiding data display device 3 is configured to display the first display area 14 and the second display area 15 on different windows. The first display area 14 and the second display area 15 can be switched through the window switching tab 8.

The configuration of the threshold setting window 7e is now described with reference to FIG. 9. The threshold setting window 7e is displayed by tapping the setting button 11 displayed on the voiding diary window 7a, the day and night voiding frequency window 7b, and the within-period total voided volume window 7c. The voiding data display device 3 is configured to allow the user to change settings of the voiding frequency, the voided volume, the voiding interval, and the time regarded as the night time after waking up on the threshold setting window 7e. The threshold setting window 7e is an example of a "threshold input receiver" in the claims.

### Advantages of This Embodiment

According to this embodiment, the following advantages are obtained.

According to this embodiment, as described above, the voiding data display device 3 includes the voiding data display controller 5a configured or programmed to display the voiding data including the voiding date and time and the voided volume, the time data display controller 5b configured or programmed to display the data on a wake-up time and the data on a fall-asleep time, and the analysis result display controller 5c configured or programmed to display the results of analysis of the voiding data, and is configured to display the voiding data, the data on a wake-up time, the data on a fall-asleep time, and the results of analysis of the voiding data on the same window. Accordingly, data necessary for diagnosis, such as the voiding data, the data on a wake-up time, the data on a fall-asleep time, and the results of analysis of the voiding data, can be displayed on the same window. Consequently, when diagnosing patient's voiding data, the doctor can link patient's lifestyle habits, such as waking up and falling asleep, to the patient's voiding data and easily grasp the same.

According to this embodiment, as described above, the voiding data display device 3 is configured to visually distinguishably display the first voiding data logged from the wake-up time to the fall-asleep time and the second voiding data logged from the fall-asleep time to the wake-up time in the different display modes. Accordingly, the voiding data during waking up can be visually distinguished from the voiding data during falling asleep, and thus the doctor can determine whether the voiding data is data during waking up or data during falling asleep at a glance without comparing the voiding time with the wake-up time and the fall-asleep time.

According to this embodiment, as described above, the voiding data display device 3 is configured to display the first voiding data logged from the wake-up time to the fall-asleep time (the voiding data in the range of the area 20 representing "during waking up ") and the second voiding data logged from the fall-asleep time to the wake-up time (the voiding data in the range of the area 21 representing "during falling asleep") in character colors different from each other. Accordingly, the first voiding data logged from the wake-up time to the fall-asleep time and the second voiding data logged from the fall-asleep time to the wake-up time are color-coded, and thus it is possible to more easily distinguish between the voiding data during waking up and the voiding data during falling asleep.

According to this embodiment, as described above, the voiding data display device 3 includes the first display area 14 arranged for displaying the data during the unit time period including the voiding data, the data on a wake-up time, the data on a fall-asleep time, and the results of analysis of the voiding data, and the second display area 15 arranged for displaying at least one of the data included in the first display area 14 over the plurality of unit time periods. Accordingly, for example, the presence or absence of a sudden, abnormal change in a short period of time can be checked from the data during the unit time period including the voiding data, the data on a wake-up time, the data on a fall-asleep time, and the results of analysis of the voiding data. In addition, long-term progress such as the progress of treatment can be checked from the data over the plurality of unit time periods including the voiding data, the data on a wake-up time, the data on a fall-asleep time, and the results of analysis of the voiding data.

According to this embodiment, as described above, the first display area 14 is arranged for displaying the data for one day as a unit time period in a time sequence, and the second display area 15 is arranged for graphically displaying the data for multiple days. Accordingly, in the first display area 14, actual data in a time sequence of one day can be checked, and thus an abnormal change or the like in one day can be checked in more detail. Moreover, in the second display area 15, the data for multiple days can be graphically and visually checked, and thus the progress of treatment or the like can be more easily grasped.

According to this embodiment, as described above, the voiding data display device 3 is configured to display the first display area 14 and the second display area 15 on the different windows. Accordingly, too much stuffing of information on one window can be significantly reduced or prevented, and thus it is possible to make the voiding data easy to see.

According to this embodiment, as described above, the results of analysis of the voiding data include the total voided volume, the total daily voiding frequency, the average voided volume during waking up, the voiding frequency during waking up, the average voided volume during falling asleep, and the voiding frequency during falling asleep. Accordingly, the results of analysis of the voiding data necessary for diagnosis, such as the total voided volume, the total daily voiding frequency, the average voided volume during waking up, the voiding frequency during waking up, the average voided volume during falling asleep, and the voiding frequency during falling asleep, can be checked along with the voiding data, the data on a wake-up time, and the data on a fall-asleep time, and thus the burden of diagnosis can be reduced.

According to this embodiment, as described above, the voiding data display device 3 further includes the additional information display controller 5d configured or programmed to display the additional information of the voiding data including urinary urgency, urine leakage, and the presence or absence of defecation. Accordingly, the situation at the time of voiding can be grasped in more detail, and thus the accuracy of diagnosis can be improved.

According to this embodiment, as described above, the time data display controller 5b includes the time data display controller 5b configured or programmed to display, between the voiding data arranged in a time sequence, the wake-up time indication 12 and the fall-asleep time indication 13 as the data on a wake-up time and the data on a fall-asleep time, respectively. Accordingly, the wake-up time and the fall-asleep time can be visually grasped through the wake-up time indication 12 and the fall-asleep time indication 13, and thus it is possible to easily distinguish between the range of the voiding data during waking up and the range of the voiding data during falling asleep among the voiding data arranged in a time sequence.

According to this embodiment, as described above, the voiding data display device 3 further includes the highlighting controller 5f configured or programmed to display the indication 22 that highlights the numerical data exceeding the threshold among the voiding data and the results of analysis of the voiding data. Accordingly, an oversight of data indicating an abnormal value (a value exceeding the threshold set by the doctor) can be significantly reduced or prevented.

According to this embodiment, as described above, the voiding data display device 3 further includes the threshold input receiver 70 configured to receive a threshold input, and the highlighting controller 5f is configured or programmed to be able to change the threshold upon the received threshold input. Accordingly, the doctor as a user can freely set an appropriate threshold, and thus the convenience of diagnosis in accordance with the criteria different for each doctor can be improved.

According to this embodiment, as described above, the voiding data display device 3 further includes the wake-up and fall-asleep time input receiver 71 configured to receive inputs of the data on a wake-up time and the data on a fall-asleep time, and the wake-up and fall-asleep time modifier 5g configured to modify the data on a wake-up time and the data on a fall-asleep time based on the input data acquired by the wake-up and fall-asleep time input receiver 71. Accordingly, the data on a wake-up time and the data on a fall-asleep time can be automatically acquired. When the acquired data is incorrect, it can be modified. Consequently, it is possible to avoid making a diagnosis using erroneous data, and thus the accuracy of the diagnosis can be improved.

According to this embodiment, as described above, the voiding data display system 100 includes the voiding data display device 3 including the voiding data display controller 5a configured or programmed to display the voiding data including the voiding date and time and the voided volume, the time data display controller 5b configured or programmed to display the data on a wake-up time and the data on a fall-asleep time, and the analysis result display controller 5c configured or programmed to display the results of analysis of the voiding data, and the voided volume measurement device 1 wirelessly connectable to the voiding data display device 3. Furthermore, the voiding data display device 3 is configured to acquire the voiding data, the data on a wake-up time, and the data on a fall-asleep time from the voided volume measurement device 1. Accordingly, the voiding data display device 3 can acquire data necessary for the doctor to make a diagnosis from the voided volume measurement device 1, and display the voiding data, the data on a wake-up time, the data on a fall-asleep time, and the results of analysis of the voiding data. Consequently, when diagnosing the patient's voiding data, the doctor can easily grasp the patient's condition. In addition, the voiding data can be acquired from the voided volume measurement device 1, and thus it is possible to save the doctor the hassle of manual entry. Furthermore, analyzed data can be acquired, and thus it is possible to save the doctor the hassle of analysis.

### Modified Examples

The embodiment disclosed this time must be considered as illustrative in all points and not restrictive. The scope of the present invention is not shown by the above description of the embodiment but by the scope of claims for patent, and all modifications (modified examples) within the meaning and scope equivalent to the scope of claims for patent are further included.

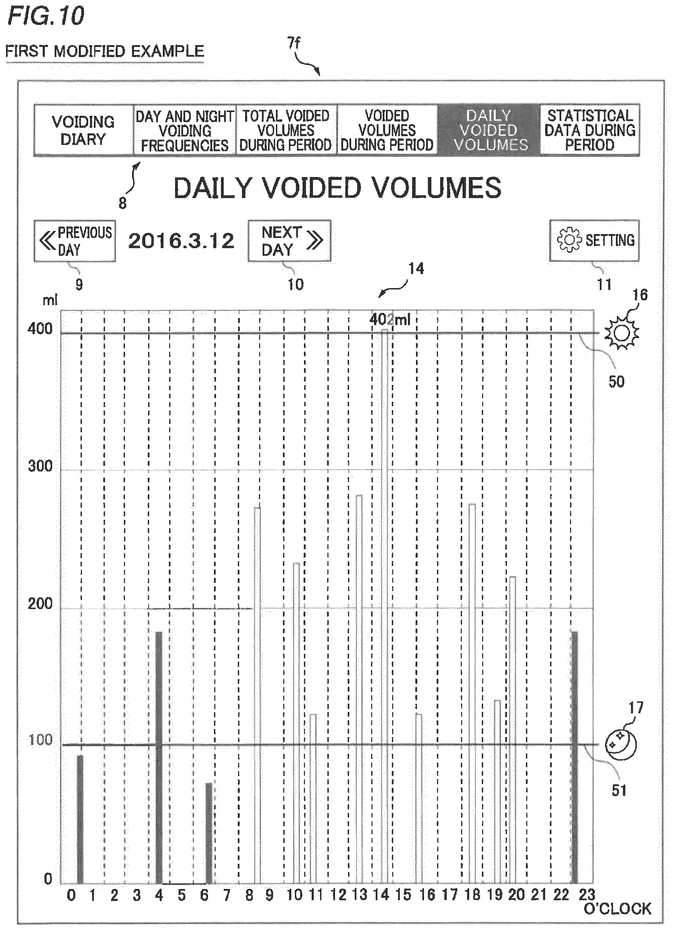

For example, while the example in which when the data for one day as a unit time period is displayed in a time sequence, the data is displayed in a tabular form has been shown in the aforementioned embodiment, the present invention is not limited to this. For example, as on a daily voided volume window 7f shown in FIG. 10, a daily voided volume may be graphically displayed. According to this configuration, voiding times, voided volumes, voiding intervals, etc. can be graphically and intuitively grasped.

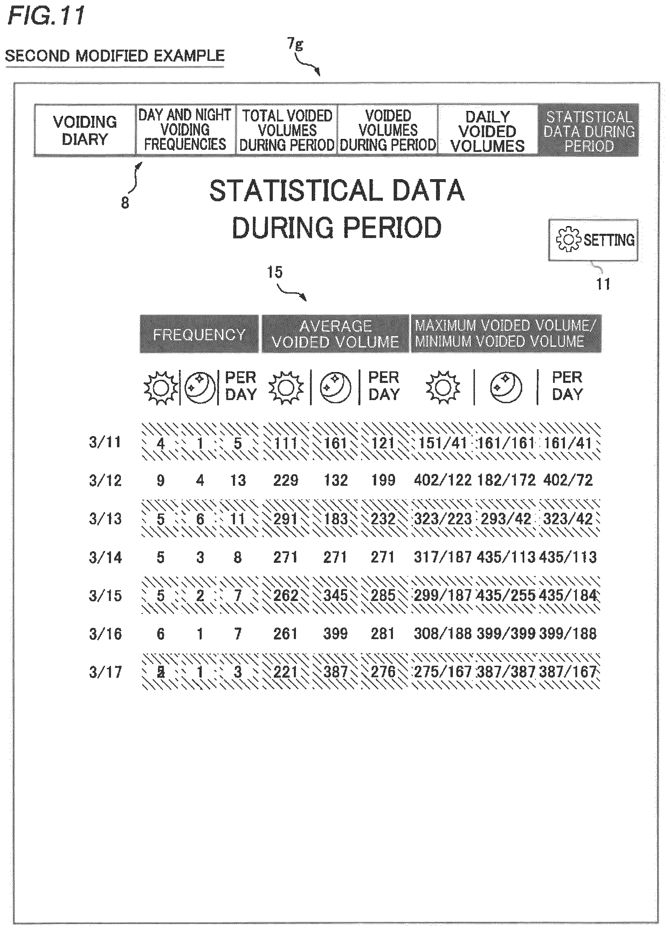

While the example in which the data for multiple days is graphically displayed in the second display area 15 has been shown in the aforementioned embodiment, the present invention is not limited to this. For example, as on a within-period statistical data window 7g shown in FIG. 11, data for multiple days may be displayed in a tabular form.

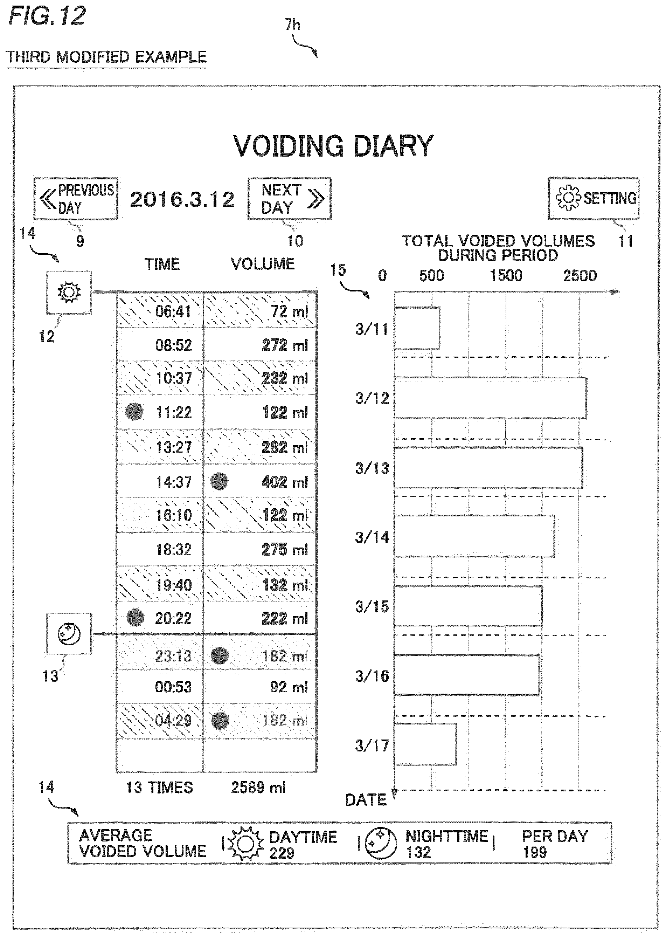

While the example in which the first display area 14 and the second display area 15 are displayed on the different windows by switching through the window switching tab 8 has been shown in the aforementioned embodiment, the present invention is not limited to this. For example, as on a voiding diary window 7h shown in FIG. 12, a first display area 14 and a second display area 15 may be displayed on the same window. On the voiding diary window 7h, voiding data during a unit time period, data on a wake-up time, data on a-fall-asleep time, and results of analysis are displayed in the first display area 14 while data on total voided volumes over a plurality of unit time periods is graphically displayed in the second display area 15.

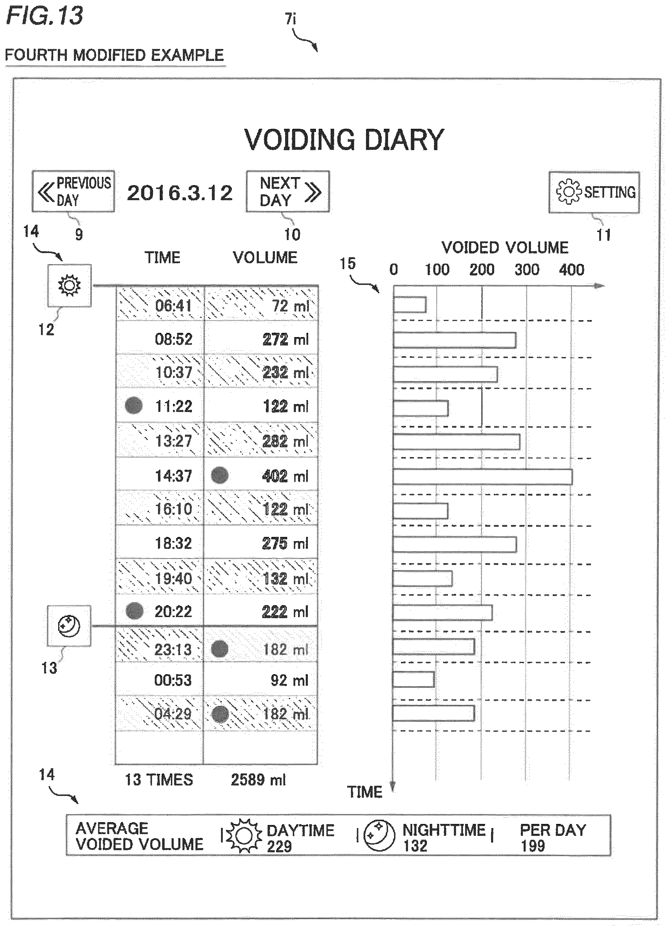

Alternatively, as on a voiding diary window 7i shown in FIG. 13, voided volumes during a unit time period may be displayed in a second display area 15.

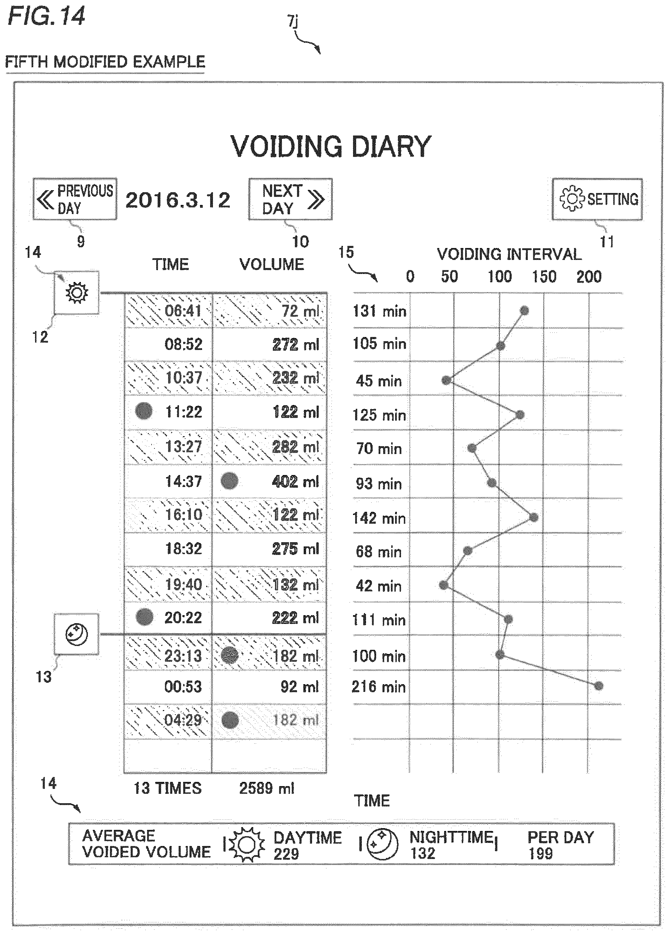

Alternatively, as on a voiding diary window 7j shown in FIG. 14, voiding intervals during a unit time period may be graphically displayed in a second display area 15. In the second display area 15, any data corresponding to the day and night voiding frequency window 7b to the within-period voided volume window 7d as shown in FIGS. 6 to 8 may be displayed.

While the example in which the voiding data during waking up and the voiding data during falling asleep are visually distinguishably displayed by making the character color of the voiding data during waking up different from the character color of the voiding data during falling asleep has been shown in the aforementioned embodiment, the present invention is not limited to this. For example, the voiding data during waking up and the voiding data during falling asleep may be displayed in such a manner that the background color of the voiding data during waking up and the background color of the voiding data during falling asleep are different from each other.

While the example in which the indication 22 is displayed next to the voiding data exceeding the threshold to highlight the voiding data has been shown in the aforementioned embodiment, the present invention is not limited to this. For example, instead of the indication, the numerical value of the voiding data may be made bold or blinked for highlighting.

While the example in which the voided volume measurement device 1 and the voiding data display device 3 are wirelessly connected to each other has been shown in the aforementioned embodiment, the present invention is not limited to this. The voiding data display device 3 may be configured to be connected to the voided volume measurement device 1 by wire, or may be configured to acquire voiding data via a storage medium such as a USB memory or an SD card.

While the example in which the voiding data display system 100 includes the weight scale 2a and the subject terminal 2b as the voided volume measurement device 1 has been shown in the aforementioned embodiment, the present invention is not limited to this. Any measurement device may be used as long as the same can measure the voided volume of the subject and transmit the voiding data to the voiding data display device 3.

While the example in which the analysis result display controller 5c analyzes the voiding data and displays the analyzed voiding data on the display 7 has been shown in the aforementioned embodiment, the present invention is not limited to this. For example, the results of analysis of the voiding data analyzed by the voided volume measurement device 1 may be acquired and displayed, or the results of analysis of the voiding data analyzed by the doctor or the like may be acquired and displayed.

While the example in which the voiding data display device 3 is a tablet terminal has been shown in the aforementioned embodiment, the present invention is not limited to this. For example, the voiding data display device 3 may be a personal computer (PC).

While the example in which the wake-up and fall-asleep indication display controller 5e displays the wake-up time indication 12 and the fall-asleep time indication 13 as the data on a wake-up time and the data on a fall-asleep time has been shown in the aforementioned embodiment, the present invention is not limited to this. For example, the time data display controller may be configured to display a wake-up time and a fall-asleep time.

While the example in which data is displayed defining one day, which serves as a unit time period, as a period from waking up to waking up the next day has been shown in the aforementioned embodiment, the present invention is not limited to this. Data display of one day may be divided at 0 o'clock to 24 o'clock.

### Description of Reference Numerals

1: voided volume measurement device
3: voiding data display device
5a: voiding data display controller
5b: time data display controller
5c: analysis result display controller
5d: additional information display controller
5e: wake-up and fall-asleep indication display controller
5f: highlighting controller
5g: wake-up and fall-asleep time modifier
12: wake-up time indication
12a: wake-up time change list (wake-up and fall-asleep time input receiver)
13: fall-asleep time indication
13a: fall-asleep time change list (wake-up and fall-asleep time input receiver)
14: first display area
15: second display area
22: indication that highlights numerical data exceeding a threshold
70: threshold input receiver
71: wake-up and fall-asleep time input receiver

## Claims

1. A voiding data display device comprising:
a voiding data display controller configured or programmed to display voiding data including a voiding date and time and a voided volume;
a time data display controller configured or programmed to display data on a wake-up time and data on a fall-asleep time; and
an analysis result display controller configured or programmed to display a result of analysis of the voiding data;
wherein the voiding data, the data on the wake-up time, the data on the fall-asleep time, and the result of the analysis of the voiding data are displayed on a same window.

2. The voiding data display device according to claim 1, configured to visually distinguishably display a first voiding data logged from the wake-up time to the fall-asleep time and a second voiding data logged from the fall-asleep time to the wake-up time in different display modes.

3. The voiding data display device according to claim 2, configured to display the first voiding data logged from the wake-up time to the fall-asleep time and the second voiding data logged from the fall-asleep time to the wake-up time in character colors different from each other or background colors different from each other.

4. The voiding data display device according to any one of claims 1 to 3, including a first display area arranged for displaying data during a unit time period including the voiding data, the data on the wake-up time, the data on the fall-asleep time, and the result of the analysis of the voiding data is displayed, and a second display area arranged for displaying at least one of the data included in the first display area is displayed over a plurality of unit time periods.

5. The voiding data display device according to claim 4, wherein
the first display area is arranged for displaying data for one day as the unit time period in a time sequence; and
the second display area is arranged for graphically displaying data for multiple days.

6. The voiding data display device according to claim 4 or 5, configured to display the first display area and the second display area on different windows.

7. The voiding data display device according to any one of claims 1 to 6, wherein the result of the analysis of the voiding data includes at least one of a total voided volume, a total daily voiding frequency, and a set of an average voided volume during waking up and an average voided volume during falling asleep and/or a set of a voiding frequency during waking up and a voiding frequency during falling asleep.

8. The voiding data display device according to any one of claims 1 to 7, further comprising an additional information display controller configured or programmed to display additional information of the voiding data including at least one of urinary urgency, urine leakage, and a presence or absence of defecation.

9. The voiding data display device according to any one of claims 1 to 8, wherein the time data display controller includes a wake-up and fall-asleep indication display controller configured or programmed to display, between the voiding data arranged in a time sequence, a wake-up time indication and a fall-asleep time indication as the data on the wake-up time and the data on the fall-asleep time, respectively.

10. The voiding data display device according to any one of claims 1 to 9, further comprising a highlighting controller configured or programmed to display an indication that highlights numerical data exceeding a threshold among at least one of the voiding data and the result of the analysis of the voiding data.

11. The voiding data display device according to claim 10, further comprising a threshold input receiver configured to receive an input of the threshold;
wherein the highlighting controller is configured or programmed to be able to change the threshold upon the received input of the threshold.

12. The voiding data display device according to any one of claims 1 to 11, further comprising:
a wake-up and fall-asleep time input receiver configured to receive inputs of the data on the wake-up time and the data on the fall-asleep time; and
a wake-up and fall-asleep time modifier configured to modify the data on the wake-up time and the data on the fall-asleep time based on input data acquired by the wake-up and fall-asleep time input receiver.

13. A voiding data display system comprising:
a voiding data display device including:
a voiding data display controller configured or programmed to display voiding data including a voiding date and time and a voided volume;
a time data display controller configured or programmed to display data on a wake-up time and data on a fall-asleep time; and
an analysis result display controller configured or programmed to display a result of analysis of the voiding data; and
a voided volume measurement device connectable to the voiding data display device wirelessly or by wire;
wherein the voiding data display device is configured to acquire at least one of the voiding data and a set of the data on the wake-up time and the data on the fall-asleep time from the voided volume measurement device.
